# EUROPEAN PATENT SPECIFICATION

(11) **EP 1 529 839 B1**
(45) Date of publication and mention of the grant of the patent: **09.04.2008**
(21) Application number: 04026719.7
(22) Date of filing: 10.11.2004
(51) Int. Cl.: C12N 9/12, C12P 13/04

(54) **Mutant phosphoribosylpyrophosphate synthetase and method for producing L-histidine**
Mutierte Phosphoribosylpyrophosphat Synthetase und Methode zur L-Histidin-Produktion
Phosphoribosylpyrophosphat synthetase mutant et methode pour la production de L-histidine

(30) Priority: 10.11.2003 RU 2003132412; 07.07.2004 RU 2004120501
(43) Date of publication of application: 11.05.2005
(73) Proprietor: Ajinomoto Co., Inc., Tokyo (JP)
(72) Inventor: Klyachko, Elena Vitalievna, Moskau 119034 (RU); Shakulov, Rustem Saidovich, Moskau 117313 (RU); Kozlov, Yuri Ivanovich, Moskau 117574 (RU)
(74) Representative: Strehl Schübel-Hopf & Partner

(56) References cited:
- 0OESSLER BLAKE J ET AL: "Human X-linked phosphoribosylpyrophosphate synthetase superactivity is associated with distinct point mutations in the PRPS1 gene" JOURNAL OF BIOLOGICAL CHEMISTRY, vol. 268, no. 35, 1993, pages 26476-26481, XP002312502 ISSN: 0021-9258
- TATIBANA M ET AL: "Mammalian phosphoribosyl-pyrophosphate synthetase." ADVANCES IN ENZYME REGULATION. 1995, vol. 35, 1995, pages 229-249, XP002312503 ISSN: 0065-2571
- HOVE-JENSEN B ET AL: "PHOSPHORIBOSYLPYROPHOSPHATE SYNTHETASE OF ESCHERICHIA-COLI PROPERTIES OF THE PURIFIED ENZYME AND PRIMARY STRUCTURE OF THE PRS GENE" JOURNAL OF BIOLOGICAL CHEMISTRY, vol. 261, no. 15, 1986, pages 6765-6771, XP002312504 ISSN: 0021-9258
- KRATH BRITTA N ET AL: "Implications of secondary structure prediction and amino acid sequence comparison of class I and class II phosphoribosyl diphosphate synthases on catalysis, regulation, and quaternary structure" PROTEIN SCIENCE, vol. 10, no. 11, November 2001 (2001-11), pages 2317-2324, XP002312505 ISSN: 0961-8368

### SEQUENCE LISTING

<110> Ajinomoto Co., Inc.
<120> MUTANT PHOSPHORIBOSYLPYROPHOSPHATE SYNTHETASE AND METHOD FOR PRODUCING L-HISTIDINE
<130>
<150> RU2003132412
   <151> 2003-11-10
<150> RU200420501
   <151> 2004-07-07
<160> 7
<170> Patentln Ver. 2.1
<210> 1
   <211> 948
   <212> DNA
   <213> Escherichia coli
<220>
   <221> CDS
   <222> (1)..(948)
<400> 1
<210> 2
   <211> 315
   <212> PRT
   <213> Escherichia coli
<400> 2
<210> 3
   <211> 29
   <212> DNA
   <213> Artificial Sequence
<220>
   <223> Description of Artificial Sequence: primer
<400> 3
   ctctctagag ccgggttcga ttagtgttc 29
<210> 4
   <211> 38
   <212> DNA
   <213> Artificial Sequence
<220>
   <223> Description of Artificial Sequence: primer
<400> 4
   ctcagatctt gcctaaggat cttctcatgc ctgatatg 38
<210> 5
   <211> 23
   <212> DNA
   <213> Artificial Sequence
<220>
   <223> Description of Artificial Sequence: primer
<400> 5
   aaagtggttg caagcttcct ctc 23
<210> 6
   <211> 23
   <212> DNA
   <213> Artificial Sequence
<220>
   <223> Description of Artificial Sequence: primer
<400> 6
   gagaggaagc ttgcaaccac ttt 23
<210> 7
   <211> 21
   <212> DNA
   <213> Artificial Sequence
<220>
   <223> Description of Artificial Sequence primer
<400> 7
   cgcgcttcaa atgaaacaga t 21

### SEQUENCE LISTING

<110> Ajinomoto Co., Inc.
<120> MUTANT PHOSPHORIBOSYLPYROPHOSPHATE SYNTHETASE AND METHOD FOR PRODUCING L-HISTIDINE
<130>
<150> RU2003132412
   <151> 2003-11-10
<150> RU200420501
   <151> 2004-07-07
<160> 7
<170> PatentIn Ver. 2.1
<210> 1
   <211> 948
   <212> DNA
   <213> Escherichia coli
<220>
   <221> CDS
   <222> (1)..(948)
<400> 1
   gtg cct gat atg aag ctt ttt gct ggt aac gcc acc ccg gaa cta gca 48
   Val Pro Asp Met Lys Leu Phe Ala Gly Asn Ala Thr Pro Glu Leu Ala
   1 5 10 15
   caa cgt att gcc aac cgc ctg tac act tca ctc ggc gac gcc gct gta 96
   Gln Arg Ile Ala Asn Arg Leu Tyr Thr Ser Leu Gly Asp Ala Ala Val
   20 25 30
   ggt cgc ttt agc gat ggc gaa gtc agc gta caa att aat gaa aat gta 144
   Gly Arg Phe Ser Asp Gly Glu Val Ser Val Gln Ile Asn Glu Asn Val
   35 40 45
   cgc ggt ggt gat att ttc atc atc cag tcc act tgt gcc cct act aac 192
   Arg Gly Gly Asp Ile Phe Ile Ile Gln Ser Thr Cys Ala Pro Thr Asn
   50 55 60
   gac aac ctg atg gaa tta gtc gtt atg gtt gat gcc ctg cgt cgt gct 240
   Asp Asn Leu Met Glu Leu Val Val Met Val Asp Ala Leu Arg Arg Ala
   65 70 75 80
   tcc gca ggt cgt atc acc gct gtt atc ccc tac ttt ggc tat gcg cgc 288
   Ser Ala Gly Arg Ile Thr Ala Val Ile Pro Tyr Phe Gly Tyr Ala Arg
   85 90 95
   cag gac cgt cgc gtc cgt tcc gct cgt gta cca atc act gcg aaa gtg 336
   Gln Asp Arg Arg Val Arg Ser Ala Arg Val Pro Ile Thr Ala Lys Val
   100 105 110
   gtt gca gac ttc ctc tcc agc gtc ggt gtt gac cgt gtg ctg aca gtg 384
   Val Ala Asp Phe Leu Ser Ser Val Gly Val Asp Arg Val Leu Thr Val
           115                 120                 125
   gat ctg cac gct gaa cag att cag ggt ttc ttc gac gtt ccg gtt gat    432
   Asp Leu His Ala Glu Gln Ile Gln Gly Phe Phe Asp Val Pro Val Asp
       130                 135                 140
   aac gta ttt ggt agc ccg atc ctg ctg gaa gac atg ctg cag ctg aat    480
   Asn Val Phe Gly Ser Pro Ile Leu Leu Glu Asp Met Leu Gln Leu Asn
   145                 150                 155                 160
   ctg gat aac cca att gtg gtt tct ccg gac atc ggc ggc gtt gtg cgt    528
   Leu Asp Asn Pro Ile Val Val Ser Pro Asp Ile Gly Gly Val Val Arg
                   165                 170                 175
   gcc cgc gct atc gct aag ctg ctg aac gat acc gat atg gca atc atc    576
   Ala Arg Ala Ile Ala Lys Leu Leu Asn Asp Thr Asp Met Ala Ile Ile
               180                 185                 190
   gac aaa cgt cgt ccg cgt gcg aac gtt tca cag gtg atgcat atc atc   624
   Asp Lys Arg Arg Pro Arg Ala Asn Val Ser Gln Val Met His Ile Ile
           195                 200                 205
   ggt gac gtt gca ggt cgt gac tgc gta ctg gtc gat gat atg atc gac    672
   Gly Asp Val Ala Gly Arg Asp Cys Val Leu Val Asp Asp Met Ile Asp
       210                 215                 220
   act ggc ggt acg ctg tgt aaa gct gct gaa gct ctg aaa gaa cgt ggt    720
   Thr Gly Gly Thr Leu Cys Lys Ala Ala Glu Ala Leu Lys Glu Arg Gly
   225                 230                 235                 240
   gct aaa cgt gta ttt gcg tac gcg act cac ccg atc ttc tct ggc aac    768
   Ala Lys Arg Val Phe Ala Tyr Ala Thr His Pro Ile Phe Ser Gly Asn
                   245                 250                 255
   gcg gcg aac aac ctg cgt aac tct gta att gat gaa gtc gtt gtc tgc    816
   Ala Ala Asn Asn Leu Arg Asn Ser Val Ile Asp Glu Val Val Val Cys
               260                 265                 270
   gat acc att ccg ctg agc gat gaa atc aaa tca ctg ccg aac gtg cgt   864
   Asp Thr Ile Pro Leu Ser Asp Glu Ile Lys Ser Leu Pro Asn Val Arg
           275                 280                 285
   act ctg acc ctg tca ggt atg ctg gcc gaa gcg att cgt cgt atc agc     912
   Thr Leu Thr Leu Ser Gly Met Leu Ala Glu Ala Ile Arg Arg Ile Ser
       290                 295                 300
   aac gaa gaa tcg atc tct gcc atg ttc gaa cac taa                   948
   Asn Glu Glu Ser Ile Ser Ala Met Phe Glu His
   305                 310                 315
<210> 2
   <211> 315
   <212> PRT
   <213> Escherichia coli
<400> 2
<210> 3
   <211> 29
   <212> DNA
   <213> Artificial Sequence
<220>
   <223> Description of Artificial Sequence: primer
<400> 3
   ctctctagag ccgggttcga ttagtgttc 29
<210> 4
   <211> 38
   <212> DNA
   <213> Artificial Sequence
<220>
   <223> Description of Artificial Sequence: primer
<400> 4
   ctcagatctt gcctaaggat cttctcatgc ctgatatg 38
<210> 5
   <211> 23
   <212> DNA
   <213> Artificial Sequence
<220>
   <223> Description of Artificial Sequence: primer
<400> 5
   aaagtggttg caagcttcct ctc 23
<210> 6
   <211> 23
   <212> DNA
   <213> Artificial Sequence
<220>
   <223> Description of Artificial Sequence: primer
<400> 6
   gagaggaagc ttgcaaccac ttt 23
<210> 7
   <211> 21
   <212> DNA
   <213> Artificial Sequence
<220>
   <223> Description of Artificial Sequence primer
<400> 7
   cgcgcttcaa atgaaacaga t 21

## Description

### Technical field

The present invention relates to biotechnology, specifically to a method for producing L-amino acid, such as L-histidine. More specifically, the present invention concerns a use of new feedback-resistant enzyme involved in the biosynthesis of purines and L-histidine. More specifically, the present invention concerns a new feedback-resistant mutant phosphoribosylpyrophosphate synthetase (PRPP synthetase) from *E. coli,* bacteria of *Enterobacteriaceae* family, harboring the enzyme and a method for producing L-histidine by fermentation using the strains of bacteria.

### Background art

Conventionally the L-amino acids have been industrially produced by method of fermentation utilizing strains of microorganisms obtained from natural sources or mutants of the same especially modified to enhance L-amino acid productivity.

There have been disclosed many techniques to enhance L-amino acid productivity, for example, by transformation of microorganism by recombinant DNA (see, for example, US patent No. 4,278,765). These techniques are based on the increasing of activities of the enzymes involved in amino acid biosynthesis and/or desensitizing the target enzymes from the feedback inhibition by produced L-amino acid (see, for example, Japanese Laid-open application No. 56-18596 (1981), WO 95/16042 or US patent Nos. 5,661,012 and 6,040,160).

5-Phosphoribosyl-α-1-pyrophosphate (phosphoribosylpyrophosphate, PRPP) and adenosine-5'-triphosphate (ATP) are the initial substrates of histidine biosynthesis. PRPP tie the histidine biosynthesis into divergent pathway with the biosynthesis of pyrimidine nucleotides, purine nucleotides, pyridine nucleotides, and tryptophan *(*Escherichia coli and Salmonella, Second Edition, Editor in Chief: F.C. Neidhardt, ASM Press, Washington D.C., 1996).

Many nucleotides inhibit activity of PRPP synthetase competitively with ATP. However, the only potent nucleotide inhibitor is adenosine-5'-diphosphate (ADP); it competes with ATP and is an allosteric inhibitor that binds to a site different from the active site (Hove-Jensen, B. et al, J. Biol. Chem. 261:6765-6771 (1986).

Mutants with altered PRPP synthetase have been obtained in both *E. coli* and *S. typhimurium.* One of the *E. coli* mutants produces a PRPP synthetase with a 27-fold increase in the Kₘ value for ATP, and the enzyme is no longer inhibited by AMP. This mutation results from substitution of aspartate 128 by alanine (*prsDA* mutation). One *S. typhimurium prs* mutant is temperature sensitive and has only 20% of the wild-type PRPP synthetase activity. This mutant enzyme had elevated Kₘ values for ATP and ribose 5-phosphate and reduced sensitivity to inhibition by ADP. The mutation is the result of the replacement of arginine 78 by cysteine (Escherichia coli and Salmonella, Second Edition, Editor in Chief: F.C. Neidhardt, ASM Press, Washington D.C., 1996).

It is well known that superactivity of human PRPP synthetase and resistance to purine nucleotide are associated with neurodevelopmental abnormalities in addition to hyperuricemia and gout (Becker M.A. et al, Arthritis Rheum, 18:6 Suppl: 687-94 (1975); Zoref E. et al, J. Clin. Invest., 56(5): 1093-9 (1975)). Uric acid overproduction in individuals with superactivity of PRPP synthetase results from increased production of PRPP and consequent acceleration of purine nucleotide synthesis *de novo.* It was shown that superactivity of PRPP synthetase is a result of A to G mutation at nucleotide 341 of mutant gene corresponding to asparagine to serine substitution at amino acid residue 113 of mature enzyme. Such mutant PRPP synthetase is resistant to purine nucleotides that inhibit the normal enzyme by mechanism that is noncompetitive in respect to ATP (Roessler, B.J. et al. J. Biol. Chem., v. 268, No 35, 26476-26481 (1993); Becker, M.A. et al, J. Clin. Invest., 96(5): 2133-41 (1995)).

A process for producing purine nucleosides via fermentation of microorganism belonging to the genus *Escherichia* and having purine nucleoside-producing ability harboring *prsDA* mutation is disclosed (European patent application EP1004663A1). But at present there are no reports describing mutant bacterial PRPP synthetase feedback resistant to purine nucleotides and the use of such mutant PRPP synthetase for improvement of L-histidine production using L-histidine producing strains.

Roessler Blake J. et al.: "Human X-linked phosphoribosylpyrophosphate synthetase superactivity is associated with distinct point mutations in the PRPS1 gene" Journal of Biological Chemistry, vol. 268, no. 35, 1993, pages 26476-26481, XP002312502 ISSN: 0021-9258, disclose that a mutant form of human PRPP having an Asp-Ser substitution at amino acid position 113 of the protein shows resistance to feedback inhibition by purine nucleotides.

Tatibana M. et al: "Mammalian phosphoribosyl-pyrophosphate synthetase", Advances in Enzyme Regulation, 1995, vol. 35, pages 229-249, XP002312503 ISSN : 0065-2571 disclose that position 113 of mammalian PRPP corresponds to position 115 in E. coli.

### Disclosure of the invention

An object of the present invention is to provide new mutant bacterial PRPP synthetase, to develop the L-histidine producing strain with enhanced productivity of L-histidine and to provide a method for producing L-histidine using the strain.

This aim was achieved by constructing new mutant PRPP synthetase from *E. coli.* Based on the high conservatism of *prsA* gene (Taira M. et al., J. Biol. Chem., v. 262, No 31, pp.14867-14870 (1987)), the mutant PRPP synthetase from *E. coli* having mutation corresponding to the human mutation Asn-113 was constructed. It was shown that the use of such mutant PRPP synthetase could enhance L- histidine production when its additional copies are introduced into the cells of the L-histidine producing strain. Thus the present invention has been completed.

The present inventions are as follows:
1). A mutant bacterial phosphoribosylpyrophosphate synthetase (PRPP synthetase), wherein the aspartate residue corresponding to position 115 in a wild type phosphoribosylpyrophosphate synthetase from *Escherichia coli* is substituted by a serine residue, and feedback inhibition by purine nucleotides is desensitized;
2). A DNA coding for the mutant PRPP synthetase according to 1),
3). A bacterium of *Enterobacteriaceae* family, which harbors the DNA according to 2) and has an ability to produce L-histidine;
4). The bacterium according to 3), wherein activity of the mutant PRPP synthetase is enhanced;
5). The bacterium according to 4), wherein the bacterium belongs to the genus *Escherichia;*
6). The bacterium according to 4), wherein the activity of the mutant PRPP synthetase is enhanced by increasing expression amount of the mutant PRPP synthetase gene;
7). The bacterium according to 6), wherein the activity of the mutant PRPP synthetase is increased by increasing copy number the mutant PRPP synthetase gene or modifying an expression control sequence of the gene so that the expression of the gene should be enhanced;
8). The bacterium according to 7), wherein the copy number is increased by integration of additional copies of the mutant PRPP synthetase gene into chromosome of the bacterium;
9). A method for producing L-histidine, which comprises cultivating the bacterium according to 3) to 8) in a culture medium to produce and accumulate L-histidine in the culture medium, and collecting the L-histidine from the culture medium;
10). The method according to 9), wherein the bacterium has enhanced expression of genes for histidine biosynthesis.

The PRPP synthetase having a substitution at the aspartate residue corresponding to the position 115 of wild type PRPP synthetase may be referred to as "the mutant PRPP synthetase ", a DNA coding for the mutant PRPP synthetase may be referred to as "the mutant *prsA* gene" or "mutant PRPP synthetase gene", and a PRPP synthetase without the substitution may be referred to as "a wild type PRPP synthetase".

Further, the present invention will be explained in detail.

### <1> Mutant PRPP synthetase and mutant prsA gene.

It is known that the genetic and functional basis of superactivity of human PRPP synthetase associated with resistance to purine nucleotide is caused by single base substitution in *prsA* gene (Roessler, B.J. et al. J. Biol. Chem., v. 268, No 35, 26476-26481 (1993)). Based on the high conservatism of *prsA* gene (Taira M. et al., J. Biol. Chem., v. 262, No 31, pp.14867-14870 (1987)), the mutant PRPP synthetase from *E. coli* having mutation corresponding to the human mutation Asn-113 was constructed. Such mutation is unknown for all bacterial PRPP synthetases. Term "bacterial PRPP synthetase" means the PRPP synthetase existing in the bacteria of *Enterobacteriaceae* family, corynebacteria, bacteria belonging to the genus *Bacillus* etc. *Enterobacteriaceae* family includes bacteria belonging to the genera *Escherichia, Erwinia, Providencia* and *Serratia.* The genus *Escherichia* is preferred.

The substitutions of the amino acid residue corresponding to the aspartate at position 115 of PRPP synthetase of *Escherichia coli* with any amino acid, preferably with serine, in the amino acid sequence of wild type PRPP synthetase [EC 2.7.6.1] from *E. coli* leads to formation of a mutant protein feedback resistant to purine nucleotides, such as purine di- and mononucleotides, mainly guanosine-5'-diphosphate (GDP), adenosine-5'-diphosphate (ADP) and adenosine-5'-monophosphate (AMP).

The mutant PRPP synthetase is obtained based on the sequences by introducing mutations into a wild type *prsA* gene using ordinary methods. As a wild type *prsA* gene, the *prsA* gene of *E. coli* can be mentioned (nucleotide numbers 1260151 to 1261098 in the sequence of GenBank Accession NC_000913, gi:16129170, SEQ ID NO: 1). The *prsA* gene is located between *ychM* and *ychB* ORFs on the chromosome of *E. coli* strain K-12. Therefore, *prsA* gene can be obtained by PCR (polymerase chain reaction; refer to White, T.J. et al., Trends Genet., 5, 185 (1989)) utilizing primers prepared based on the nucleotide sequence of the gene. Genes coding for PRPP synthetase of other microorganisms can be obtained in a similar manner.

Term "activity of PRPP synthetase" means activity to catalyze the reaction of formation 5-phosphoribosyl-α-1-pyrophosphate (PRPP) from ribose-5-phosphate and ATP with release of AMP. The PRPP synthetase activity of the extracts and degrees of inhibition by ADP can be measured using the partially modified method of K. F. Jensen et al. (Analytical Biochemistry, 98, 254-263 (1979)). Specifically, [α-³²P]ATP can be used as the substrate and [³²P]AMP produced by the reaction should be measured.

In the present invention, "L-amino acid residue corresponding to position 115" means an aspartate residue corresponding to the amino acid residue of position 115 in the amino acid sequence of SEQ ID NO: 2.

A position of amino acid residue may change. For example, if an amino acid residue is inserted at N-terminus portion, the amino acid residue inherently locates at the position 115 becomes position 116. In such a case, the amino acid residue corresponding to the original position 115 is designated as the amino acid residue at the position 115 in the present invention.

To determine L-amino acid residue corresponding to position 115 of PRPP synthetase from *E. coli,* it is necessary to make alignment of the amino acid sequence of PRPP synthetase from *E. coli* (SEQ ID NO: 2) and an amino acid sequence of PRPP synthetase from bacterium of interest, and define the L-amino acid designate by number 115 in the PRPP synthetase from bacterium of interest.

The DNA, which codes for the mutant PRPP synthetase, can be obtained by isolating a DNA which hybridizes with DNA having known *prsA* gene sequence or part of it as a probe under stringent conditions, and which codes for a protein having the PRPP synthetase activity, from a cell harboring the mutant PRPP synthetase which is subjected to mutation treatment.

The term "stringent conditions" referred to herein means a condition under which so-called specific hybrid is formed, and non-specific hybrid is not formed. It is difficult to express this condition precisely by using any numerical value. However, for example, the stringent conditions include a condition under which DNAs having high homology, for example, DNAs having homology of not less than 50% with each other are hybridized, and DNAs having homology lower than the above with each other are not hybridized.

To evaluate degree of protein or DNA homology several calculation methods, such as BLAST search, FASTA search and CrustalW, can be used.

BLAST (Basic Local Alignment Search Tool) is the heuristic search algorithm employed by the programs blastp, blastn, blastx, megablast, tblastn, and tblastx; these programs ascribe significance to their findings using the statistical methods of Karlin, Samuel and Stephen F. Altschul ("Methods for assessing the statistical significance of molecular sequence features by using general scoring schemes". Proc. Natl. Acad. Sci. USA, 1990, 87:2264-68; "Applications and statistics for multiple high-scoring segments in molecular sequences". Proc. Natl. Acad. Sci. USA, 1993, 90:5873-7). FASTA search method is described by W. R. Pearson ("Rapid and Sensitive Sequence Comparison with FASTP and FASTA", Methods in Enzymology, 1990 183:63- 98). ClustalW method is described by Thompson J.D., Higgins D.G. and Gibson T.J. ("CLUSTAL W: improving the sensitivity of progressive multiple sequence alignment through sequence weighting, position-specific gap penalties and weight matrix choice", Nucleic Acids Res. 1994, 22:4673-4680).

Alternatively, the stringent condition is exemplified by a condition under which DNA's are hybridized with each other at a salt concentration corresponding to an ordinary condition of washing in Southern hybridization, i.e., 60 °C, 1 x SSC, 0.1 % SDS, preferably 0.1 x SSC, 0.1 % SDS. As a probe for the DNA that codes for variants and hybridizes with *prsA* gene, a partial sequence of the nucleotide sequence of SEQ ID NO: 1 can also be used. Such a probe may be prepared by PCR using oligonucleotides produced based on the nucleotide sequence of SEQ ID NO: 1 as primers, and a DNA fragment containing the nucleotide sequence of SEQ ID NO: 1 as a template. When a DNA fragment in a length of about 300 bp is used as the probe, the conditions of washing for the hybridization consist of, for example, 50 °C, 2 x SSC, and 0.1% SDS. Duration of washing procedure depends on the type of membrane used for blotting and, as a rule, is recommended by manufacturer. For example, recommended duration of washing the Hybond^{™} N+ nylon membrane (Amersham) in the stringent conditions is 15 minutes.

The gene, which is hybridizable under the condition as described above, includes those having a stop codon generated within a coding region of the gene, and those having no activity due to mutation of active center. However, such inconveniences can be easily removed by ligating the gene with a commercially available expression vector, and investigating PRPP synthetase activity of expressed protein.

### <2> Bacterium of the present invention.

The bacterium of the present invention is an L-histidine producing bacterium of *Enterobacteriaceae* family harboring DNA coding for mutant PRPP synthetase of the present invention. Further, the bacterium of the present invention is an L-histidine producing bacterium of *Enterobacteriaceae* family having increased activity of mutant PRPP synthetase of the present invention. Concretely, the bacterium of the present invention is an L-histidine producing bacterium of *Enterobacteriaceae* family, wherein L-histidine production by the bacterium is enhanced by enhancing an activity of the protein of the present invention in a cell of the bacterium. Concretely, the bacterium of the present invention is an L-histidine producing bacterium belonging to the genus *Escherichia,* wherein L-histidine production by the bacterium is enhanced by enhancing an activity of the protein of the present invention, namely mutant PRPP synthetase, in a cell of the bacterium. More concretely, the bacterium of present invention harbors the DNA having mutant *prsA* gene over-expressed in the chromosome or in a plasmid in the bacterium and has enhanced ability to produce L-histidine.

"Bacterium, which has an ability to produce L-histidine" means a bacterium, which has an ability to accumulate L-histidine in a medium, when the bacterium of the present invention is cultured in the medium. The L-histidine producing ability may be imparted or enhanced by breeding. The term "bacterium, which has an ability to produce L-histidine" used herein also means a bacterium, which is able to produce and accumulate L-histidine in a culture medium in amount larger than a wild type or parental strain, and preferably means that the microorganism is able to produce and accumulate in a medium an amount of not less than 0.5 g/L, more preferably not less than 1.0 g/L of L-histidine.

*Enterobacteriaceae* family includes bacteria belonging to the genera *Escherichia, Erwinia, Providencia* and *Serratia.* The genus *Escherichia* is preferred.

The term "a bacterium belonging to the genus *Escherichia"* means that the bacterium which is classified as the genus *Escherichia* according to the classification known to a person skilled in the microbiology. As examples of the microorganism belonging to the genus *Escherichia* used in the present invention, *Escherichia coli* (*E. coli*) can be mentioned.

The term "activity of the mutant PRPP synthetase is enhanced" means that the activity per cell has become higher than that of a non-modified strain, for example, a wild-type strain. For example, there can be mentioned a case where number of the mutant PRPP synthetase molecules per cell increases, a case where specific activity per mutant PRPP synthetase molecule increases and so forth. Further, as a wild-type strain that serves as an object for comparison, for example, the *Escherichia coli* K-12 can be mentioned. As a result of enhancement of intracellular activity of the mutant PRPP synthetase, an effect that the amount of L-histidine accumulation in a medium increases is observed.

Enhancement of the mutant PRPP synthetase activity in a bacterial cell can be attained by enhancement of expression of a gene coding for the mutant PRPP synthetase. As the mutant PRPP synthetase gene, any of genes coding for mutant PRPP synthetase derived from bacteria of *Enterobacteriaceae* family and genes derived from other bacteria such as coryneform bacteria can be used. Among these, genes derived from bacteria belonging to the genus *Escherichia* are preferred.

Transformation of bacterium with a DNA coding for a protein means introduction of the DNA into bacterium cell for example by conventional methods to increase expression of the gene coding for the protein of present invention and to enhance the activity of the protein in the bacterial cell.

The methods of the enhancement of gene expression include an increasing of the gene copy number. Introduction of a gene into a vector that is able to function in a bacterium belonging to the genus *Escherichia* increases copy number of the gene. For such purposes multi-copy vectors can be preferably used. The multi-copy vector is exemplified by pBR322, pUC19, pBluescript KS⁺, pACYC177, pACYC184, pAYC32, pMW119, pET22b or the like. Besides, enhancement of gene expression can be achieved by introduction of multiple copies of the gene into bacterial chromosome by, for example, method of homologous recombination or the like.

On the other hand, the enhancement of gene expression can be achieved by locating the DNA of the present invention under control of more potent promoter instead of the native promoter. Strength of promoter is defined by frequency of acts of the RNA synthesis initiation. Methods for evaluation the strength of promoter and an examples of potent promoters are described by Deuschle, U., Kammerer, W., Gentz, R., Bujard, H. (Promoters in Escherichia coli: a hierarchy of in vivo strength indicates alternate structures. EMBO J. 1986, 5, 2987-2994). For example, P_{R} promoter is known as a potent constitutive promoter. Other known potent promoters are P_{L} promoter, *lac* promoter, *trp* promoter, *trc* promoter, of lambda phage and the like.

The enhancement of translation can be achieved by introducing into the DNA of the present invention more efficient Shine-Dalgarno sequence (SD sequence) instead of native SD sequence where SD sequence is a region upstream of the start codon of mRNA interacting with the 16S RNA of ribosome (Shine J. and Dalgarno L., Proc. Natl. Acad. Sci. U S A, 1974, 71, 4, 1342-6).

Using the potent promoter can be combined with multiplication of gene copies.

Methods for preparation of chromosomal DNA, hybridization, PCR, preparation of plasmid DNA, digestion and ligation of DNA, transformation, selection of an oligonucleotide as a primer and the like may be ordinary methods well known to one skilled in the art. These methods are described in Sambrook, J., and Russell D., "Molecular Cloning A Laboratory Manual, Third Edition", Cold Spring Harbor Laboratory Press (2001) and the like.

The bacterium of the present invention can be obtained by introduction of the aforementioned DNAs into bacterium inherently having ability to produce L-histidine. Alternatively, the bacterium of present invention can be obtained by imparting ability to produce L-histidine to the bacterium already harboring the DNAs.

As a parent strain which is to be enhanced in activity of the protein of the present invention, bacteria belonging to the genus *Escherichia* having L-histidine producing ability, the L-histidine producing bacterium strains belonging to the genus *Escherichia,* such as *E. coli* strain 24 (VKPM B-5945, Russian patent 2003677); *E. coli* strain 80 (VKPM B-7270, Russian patent 2119536); *E. coli* strains NRRL B-12116-B12121 (US patent 4388405); *E. coli* strains H-9342 (FERM BP-6675) and H-9343 (FERM BP-6676) (US patent 6344347); *E. coli* strain H-9341 (FERM BP-6674) (European patent application 1085087A2); *E. coli* strain AI80/pFM201 (US patent 6258554) and the like can be mentioned.

It is desired that the L-histidine producing bacterium be further modified to have enhanced expression of L-histidine biosynthesis. Genes effective for L-histidine biosynthesis include *hisG* gene and genes of *hisBHAFI* operon, preferably *hisG* gene encoding ATP phosphoribosyl transferase of which feedback inhibition by L-histidine is desensitized (Russian patents 2003677 and 2119536).

### <3> Method of the present invention.

The method of present invention includes method for producing an L-histidine, comprising steps of cultivating the bacterium of the present invention in a culture medium, to allow the L-histidine to be produced and accumulated in the culture medium, and collecting the L-histidine from the culture medium.

In the present invention, the cultivation, the collection and purification of L-histidine from the medium and the like may be performed in a manner similar to the conventional fermentation method wherein an amino acid is produced using a microorganism. A medium used for culture may be either a synthetic medium or a natural medium, so long as the medium includes a carbon source and a nitrogen source and minerals and, if necessary, appropriate amounts of nutrients which the microorganism requires for growth. The carbon source may include various carbohydrates such as glucose and sucrose, and various organic acids. Depending on the mode of assimilation of the used microorganism, alcohol including ethanol and glycerol may be used. As the nitrogen source, various ammonium salts such as ammonia and ammonium sulfate, other nitrogen compounds such as amines, a natural nitrogen source such as peptone, soybean-hydrolysate and digested fermentative microorganism are used. As minerals, potassium monophosphate, magnesium sulfate, sodium chloride, ferrous sulfate, manganese sulfate, calcium chloride, and the like are used. Some additional nutrient can be added to the medium if necessary. For instance, if the microorganism requires proline for growth (proline auxotrophy) the sufficient amount of proline can be added to the medium for cultivation.

The cultivation is performed preferably under aerobic conditions such as a shaking culture, and stirring culture with aeration, at a temperature of 20 to 42 °C, preferably 37 to 40 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the accumulation of the target L-amino acid in the liquid medium.

After cultivation, solids such as cells can be removed from the liquid medium by centrifugation or membrane filtration, and then the target L-amino acid can be collected and purified by ion-exchange, concentration and crystallization methods.

### Best Mode for Carrying out the Invention

The present invention will be more concretely explained below with reference to Examples. In the Examples an amino acid is of L-configuration unless otherwise noted.

### Example 1. Cloning the wild type prsA gene from E. coli and construction the mutant prsDA and prsDS genes.

The entire nucleotide sequence of *E. coli* strain K-12 has already been determined (Science, 277, 1453-1474, 1997). Based on the reported nucleotide sequence the primers depicted in SEQ ID No. 3 (primer 1) and SEQ ID No.4 (primer 2) for amplification of *prsA* gene were synthesized. The primer 1 contains *Bgl*II recognition site introduced at the 5' thereof. The primer 2 contains *Xba*I recognition site introduced at the 5'-end thereof.

The chromosomal DNA of *E. coli* K12 used as template for PCR was prepared by an ordinary method. PCR was carried out on "Applied Biosystems GeneAmp PCR System 2400" under the following conditions: initial DNA denaturation at 95 °C for 3 min; then 30 cycles of denaturation at 95 °C for 30 sec, annealing at 60 °C for 60 sec and elongation at 72 °C for 120 sec; the final polymerization for 7 min at 72 °C using *Taq* polymerase (Fermentas, Lithuania). The obtained PCR fragment containing *prsA* gene without promoter was treated with *Bgl*II and *XbaI* and inserted under P_{R} promoter in integrative vector pMW119-P_{R} previously treated with the same enzymes. Vector pMW119-P_{R} was constructed from commercially available vector pMW119 by insertion of P_{R} promoter from phageλ and attR and attL sites necessary for further Mu-integration. Thus plasmid pMW-P_{R}-prsA was obtained.

Mutant *prsDA* gene (substitution of aspartate 128 with alanine in the PRPP synthetase coded by mutant *prsDA* gene) was obtained by PCR as described above using primers 1 (SEQ ID No. 3) and 2 (SEQ ID No. 4), and plasmid pUCprsDA as a template. Plasmid pUCprsDA described in details in the European patent application EP1004663A1. Resulted PCR product was treated with *Bgl*II and *XbaI* and inserted under P_{R} promoter in integrative vector pMW119-P_{R} previously treated with the same enzymes. Thus plasmid pMW-P_{R}-prsDA was obtained.

Mutant *prsDS* gene (substitution of aspartate 115 with serine in the PRPP synthetase coded by mutant *prsDS* gene) was constructed by two successive PCR. At the first stage two fragments of gene were synthesized using primers 1 (SEQ ID No. 3) and 3 (SEQ ID No. 5) for the first fragment and primers 2 (SEQ ID No. 4) and 4 (SEQ ID No. 6) for the second one. The chromosomal DNA of *E. coli* K12 was used as template. Then the resulted PCR products were separated by electrophoresis and eluted from gel. In the second PCR these two DNA fragments were annealed and mutant *prsDS* gene was completed. The obtained PCR fragment containing *prsDS* gene without promoter was treated with *Bgl*II and *XbaI* and inserted under P_{R} promoter in integrative vector pMW119-P_{R} previously treated with the same enzymes. Thus plasmid pMW-P_{R}-prsDS was obtained.

### Example 2. Effect of enhanced expression of purH gene on histidine production.

Three L-histidine producing plasmidless strains containing additional copies of *prsA, prsDA* or *prsDS* genes integrated into bacterial chromosome were constructed. The L-histidine producing *E. coli* strain 80 was used as a parental strain for integration of *prsA, prsDA* and *prsDS* genes into bacterial chromosome. The strain 80 has been described in Russian patent 2119536 and deposited in the Russian National Collection of Industrial Microorganisms (Russia, 113545 Moscow, 1^{st} Dorozhny proezd, 1) under accession number VRPM B-7270.

Integration of the genes into chromosome of strain 80 was performed in two steps. At the first step the histidine producing strain 80 was transformed with the helper plasmid containing replicon rep(p15A), transposase gene (genes cts62, ner, A, B from phage Mu-cts) and harbouring Tet^{R} marker. At the second stage the resulted strain was transformed with plasmid pMW-P_{R}-prsA, pMW-P_{R}-prsDA or pMW-P_{R}-prsDS. For integration of the gene into chromosome the heat-shocked cells were transferred to 1 ml of L-broth, incubated at 44 °C for 20 minutes, at 37 °C for 40 minutes, and then were spreaded onto L-agar containing 10 µg/ml of tetracycline and 100 µg/ml of ampicillin. Colonies grown within 48 hours at 30 °C were inoculated in 1 ml of L broth and incubated for 72 hours at 42 °C in tubes. About 10 colonies from every tube were checked for ampicillin and tetracycline resistance. Colonies sensitive to both antibiotics were tested for presence of additional copies of the *prs* gene in chromosome by PCR using primers 1 (SEQ ID No 3) and primer 5 (SEQ ID No 7). Primer 5 contains sequence complementary to attR site of phage Mu. For that purpose, freshly isolated colony was suspended in µl of water and then 1 µl was used in PCR. PCR conditions was following: initial DNA denaturation at 95 °C for 5 minutes; then 30 cycles of denaturation at 95 °C for 30 sec, annealing at 57 °C for 60 sec and elongation at 72 °C for 120 sec; the final polymerization at 72 °C for 7 min. A few antibiotic sensitive colonies tested contained necessary 1515 bp DNA fragment. Thus strains 80::P_{R}-prsA 80::P_{R}-prsDA and 80::P_{R}-prsDS were obtained.

For mini-jar batch-fermentation one loop of each strain grown on L-agar was transferred to L-broth and cultivated at 30 °C with rotation (140 rpm) to reach optical density of culture OD₅₄₀ ≈ 2.0. Then 25 ml of seed culture was added to 250 ml of medium for fermentation and cultivated at 29 °C for with rotation (1500 rpm). Duration of the batch-fermentation was approximately 35-40 hours. After the cultivation the amount of histidine accumulated in the medium was determined by paper chromatography. The paper was developed with a mobile phase: n-butanol : acetic acid : water = 4 : 1 : 1 (v/v). A solution of ninhydrin (0.5%) in acetone was used as a visualizing reagent.

The composition of the fermentation medium (pH 6.0) (g/l):

| | |
|---|---|
| Glucose | 100.0 |
| Mameno | 0.2 of TN |
| (NH₄)₂SO₄ | 8.0 |
| KH₂PO₄ | 1.0 |
| MgSO₄x7H₂O | 0.4 |
| FeSO₄x7H₂O | 0.02 |
| MnSO₄ | 0.02 |
| Thiamine | 0.001 |
| Betaine | 2.0 |
| L-proline | 0.8 |
| L-glutamate | 3.0 |
| L-aspartate | 1.0 |
| Adenosine | 0.1 |

Obtained data are presented in the Table 1.

**Table 1.**

| Strain | Integrated gene | DCW, g/l | Histidine, g/l | Yield per glucose |
|---|---|---|---|---|
| 80 | - | 8.4 | 16.9 | 20.40 |
| 80::P_{R}-prsA | *prsA* | 8.6 | 15.6 | 19.1 |
| 80::P_{R}-prsDA | *prsDA* | 7.3 | 15.8 | 19.7 |
| 80::P_{R}-prsDS | *prsDS* | 8.5 | 18.4 | 22.1 |

As it seen from the Table 1, the use of mutant *prsA* gene coding for PRPP synthetase feedback resistant to purine nucleotides improved histidine productivity of the *E. coli* strain 80.

## Claims

1. A mutant bacterial phosphoribosylpyrophosphate synthetase (PRPP synthetase), wherein the aspartate residue corresponding to position 115 in a wild type phosphoribosylpyrophosphate synthetase from *Escherichia coli* is substituted by a serine residue, and feedback inhibition by purine nucleotides is desensitized.

2. A DNA coding for the mutant PRPP synthetase according to claim 1.

3. A bacterium of *Enterobacteriaceae* family, which harbors the DNA according to claim 2 and has an ability to produce L-histidine.

4. The bacterium according to claim 3, wherein the activity of the mutant PRPP synthetase is enhanced.

5. The bacterium according to claim 4, wherein the bacterium belongs to the genus *Escherichia.*

6. The bacterium according to claim 4, wherein the activity of the mutant PRPP synthetase is enhanced by increasing the expression amount of the mutant PRPP synthetase gene.

7. The bacterium according to claim 6, wherein the activity of the mutant PRPP synthetase is increased by increasing the copy number of the mutant PRPP synthetase gene or modifying an expression control sequence of the gene so that the expression of the gene should be enhanced.

8. The bacterium according to claim 7, wherein the copy number is increased by integration of additional copies of the mutant PRPP synthetase gene into the chromosome of the bacterium.

9. A method for producing L-histidine, which comprises cultivating the bacterium according to any of claims 3 to 8 in a culture medium to produce and accumulate L-histidine in the culture medium, and collecting the L-histidine from the culture medium.

10. The method according to claim 9, wherein the bacterium has an enhanced expression of genes for histidine biosynthesis.

## Patentansprüche

1. Mutierte bakterielle Phosphoribosylpyrophosphat-Synthetase (PRPP-Synthetase), worin der Aspartatrest, welcher der Position 115 in einer Wildtyp-Phosphoribosylpyrophosphat-Synthetase aus *Escherichia coli* entspricht, durch einen Serinrest ersetzt ist und die Rückkopplungshemmung durch Purinnucleotide ausgeschaltet ist.

2. Für die mutierte PRPP-Synthetase nach Anspruch 1 kodierende DNA.

3. Bakterium der Familie *Enterobacteriaceae,* welches die DNA nach Anspruch 2 enthält und die Fähigkeit zur Produktion von L-Histidin hat.

4. Bakterium nach Anspruch 3, worin die Aktivität der mutierten PRPP-Synthetase verstärkt ist.

5. Bakterium nach Anspruch 4, worin das Bakterium zur Gattung *Escherichia* gehört.

6. Bakterium nach Anspruch 4, worin die Aktivität der mutierten PRPP-Synthetase durch Erhöhen der exprimierten Menge des mutierten PRPP-Synthetasegens verstärkt ist.

7. Bakterium nach Anspruch 6, worin die Aktivität der mutierten PRPP-Synthetase durch Erhöhen der Kopienzahl des mutierten PRPP-Synthetasegens oder durch Modifizieren einer Expressionskontrollsequenz des Gens, so dass die Expression des Gens verstärkt sein sollte, erhöht ist.

8. Bakterium nach Anspruch 7, worin die Kopienzahl durch Einführen zusätzlicher Kopien des mutierten PRPP-Synthetasegens in das Chromosom des Bakteriums erhöht ist.

9. Verfahren zum Herstellen von L-Histidin, welches das Kultivieren des Bakteriums nach einem der Ansprüche 3 bis 8 in einem Kulturmedium zur Produktion und Anhäufung von L-Histidin in dem Kulturmedium und das Gewinnen des L-Histidins aus dem Kulturmedium umfasst.

10. Verfahren nach Anspruch 9, worin das Bakterium eine verstärkte Expression der Gene für die Histidin-Biosynthese aufweist.

## Revendications

1. Phosphoribosylpyrophosphate-synthétase (PRPP-synthétase) bactérienne mutante dans laquelle le résidu d'aspartate correspondant à la position 115 dans une phosphoribosylpyrophosphate-synthétase de type sauvage d'*Escherichia coli* est remplacé par un résidu de sérine, et la rétro-inhibition par les nucléotides puriques est désensibilisée.

2. ADN codant pour la PRPP-synthétase mutante selon la revendication 1.

3. Bactérie de la famille des *Enterobacteriaceae,* qui héberge l'ADN selon la revendication 2 et a la capacité de produire de la L-histidine.

4. Bactérie selon la revendication 3, dans laquelle l'activité de la PRPP-synthétase mutante est amplifiée.

5. Bactérie selon la revendication 4, la bactérie appartenant au genre *Escherichia.*

6. Bactérie selon la revendication 4, dans laquelle l'activité de la PRPP-synthétase mutante est amplifiée par l'augmentation de la quantité d'expression du gène de la PRPP-synthétase mutante.

7. Bactérie selon la revendication 6, dans laquelle l'activité de la PRPP-synthétase mutante est augmentée par l'augmentation du nombre de copies du gène de la PRPP-synthétase mutante ou la modification d'une séquence de contrôle de l'expression du gène de sorte que l'expression du gène devrait être amplifiée.

8. Bactérie selon la revendication 7, dans laquelle le nombre de copies est augmenté par l'intégration de copies supplémentaires du gène de la PRPP-synthétase mutante dans le chromosome de la bactérie.

9. Procédé de production de L-histidine, qui comprend la culture de la bactérie selon l'une quelconque des revendications 3 à 8 dans un milieu de culture pour produire et accumuler de la L-histidine dans le milieu de culture, et le recueil de la L-histidine à partir du milieu de culture.

10. Procédé selon la revendication 9, dans lequel la bactérie a une expression amplifiée de gènes pour la biosynthèse de l'histidine.
